# EUROPEAN PATENT SPECIFICATION

(11) **EP 4 763 190 B1**
(45) Date of publication and mention of the grant of the patent: **07.10.2026**
(21) Application number: 24222502.7
(22) Date of filing: 20.12.2024
(51) Int. Cl.: A61K 9/00, A61K 9/50, A61K 31/381, A61K 47/10, A61K 47/18, A61K 47/22

(54) **PHARMACEUTICAL COMPOSITION COMPRISING DULOXETINE**
PHARMAZEUTISCHE ZUSAMMENSETZUNG MIT DULOXETIN
COMPOSITION PHARMACEUTIQUE COMPRENANT DE LA DULOXÉTINE

(43) Date of publication of application: 24.06.2026
(73) Proprietor: KRKA, d.d., Novo mesto, 8501 Novo mesto (SI)
(72) Inventor: GRADISEK, Aljosa, 8501 Novo mesto (SI); JURSA KSELA, Sonja, 8501 Novo mesto (SI); SENICA, Luka, 8501 Novo mesto (SI); HVALEC, Lucija, 8501 Novo mesto (SI); MEDIC, Ana, 8501 Novo mesto (SI); HODNIK, Ziga, 8501 Novo mesto (SI); HUDOVORNIK, Grega, 8501 Novo mesto (SI)
(74) Representative: Kutzenberger Wolff & Partner

(56) References cited:
- WO-A1-2024/127365
- US-A1- 2009 226 517

## Description

The invention relates to a pharmaceutical composition comprising or essentially consisting of - a core; - an active ingredient coating layer comprising Duloxetine or a physiologically acceptable salt thereof, preferably Duloxetine hydrochloride; - an intermediate coating layer; - an enteric coating layer; and - optionally, a finishing coating layer; wherein the active ingredient coating layer and the intermediate coating layer comprises a nitrite scavenger, which is ascorbic acid. The invention is set out in the appended claims.

Duloxetine (Dulsevia, ATC N06AX21) is a combined serotonin and noradrenaline reuptake inhibitor. It weakly inhibits dopamine reuptake with no significant affinity for histaminergic, dopaminergic, cholinergic and adrenergic receptors. Duloxetine dose-dependently increases extracellular levels of serotonin and noradrenaline in various brain areas of animals. Duloxetine is used to treat major depressive disorder, generalized anxiety disorder, obsessive-compulsive disorder, fibromyalgia, neuropathic pain and central sensitization. Duloxetine has a structure shown in formula (I):

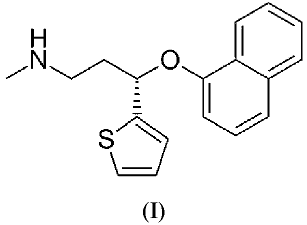

Pharmaceutical compositions comprising Duloxetine are well known in the prior art and have been described, for example in EP0693282A2, EP3335697A1, CN100362996C, CN101190208A, and CN105520920A.

Duloxetine is a secondary amine, which in the presence of nitrite can oxidize to a N-nitrosamine impurity of the active substance. When nitrosating agents such as nitrite salts under acidic conditions, react, for example, with a secondary, a tertiary or a quaternary amine, N-nitrosamines can be formed. N-nitrosamines are a class of compounds having the chemical structure of a nitroso (-N=O) group bonded to an amine. They are classified as probable human carcinogens on the basis of animal studies. Nitrites can be found in many excipients at parts per million levels, which typically come from process water, processing steps requiring acid titration, bleaching, and potentially from oxidation in air as the excipient is being heated in a drying process.

On 25 June 2020, the European Medicines Agency (EMA) issued guidance in its recommendations for the pharmaceutical industry, with the aim of reducing the content of N-nitrosamines in medicines and ensuring patient safety. As of 1 December 2022, the annex to the aforementioned guidance (Appendix 1 - Acceptable intakes established for N-nitrosamines) was updated with a safe limit for N-nitroso-Duloxetine of 100 ng/day, which corresponds to 0.83 ppm of N-nitrosamine impurities in the product.

Because of the above mentioned recommendations from EMA there is an increased need for Duloxetine products that comply with new guidelines for the amount of N-nitrosamine impurities in a pharmaceutical product and are at the same time providing a good stability.

WO2024165666A1 refers to a method for reducing the amount of nitrosating agents and/or N-nitrosamine impurities in pharmaceutically acceptable excipients. The method comprises a pretreatment step of mixing one or more pharmaceutically acceptable excipients with at least one reducing agent.

WO2024127365A1 relates to a stable multiparticulate pharmaceutical composition comprising an amine drug or a pharmaceutically acceptable salt thereof and a stabilizing amount of at least one nitrite quencher. The nitrite quencher is selected from ascorbic acid or salt thereof, amino acids selected from cysteine or salt thereof, histidine or salt thereof, glycine or salt thereof, arginine or salt thereof.

CN115721724A refers to a pharmaceutical composition, characterized in that it comprises a secondary amine compound and a pharmaceutically acceptable acid. CN115721724A also refers to the content of N-nitrosamine impurities in the pharmaceutical composition. A secondary amine can be Duloxetine and the pharmaceutically acceptable acid can be ascorbic acid.

CN114739774A refers to an antioxidant combination in the preparation of a quality control product and/or a calibrator for monitoring 78 neuropsychiatric drugs at the same time, wherein the antioxidant combination is composed of ascorbic acid, butylated hydroxytoluene, and ethylenediaminetetraacetic acid.

There is a need for improved pharmaceutical compositions of Duloxetine, in particular entericcoated capsules, which ensure safe levels of N-nitrosamine impurities and good stability during shelf life of the compositions.

It is an object of the invention to provide pharmaceutical compositions and pharmaceutical dosage forms that have improved safety, preferably because they do not contain excessive quantities of N-nitrosamines, while at the same time providing good stability.

This object has been achieved by the subject-matter of the patent claims.

It has been found that some excipients such as talc and gelatin capsules can be potentially problematic in light of increasing N-nitrosamine impurities in pharmaceutically compositions and dosage forms of Duloxetine, because these excipients can contain certain amounts of nitrites.

It has been surprisingly found that the amount of nitrite contained in gelatin capsules prior to encapsulation has an influence on the amount of N-nitrosamine impurity in the final product directly after preparation. While gelatin capsules typically are not in direct contact with the Duloxetine, the content of N-nitrosamine impurity in the dosage form can be increased after encapsulation, compared to the composition, preferably pellets, before encapsulation.

Further, it has been surprisingly found that the amount of nitrite contained in gelatin capsules prior to encapsulation has an influence on the amount of N-nitrosamine impurity in the final product after stressed conditions. Thus, a lower nitrite content in the capsule can lead to a lower amount of N-nitrosamine impurity in the final product after stressed conditions.

Still further, it has been surprisingly found that despite the use of excipients with low nitrite contents, the contents of the N-nitrosamine impurity in the dosage forms, preferably capsules, can be already close to the safe limit (0.83 ppm) at the initial point, and after stressed conditions they can exceed the safe limit even under milder conditions. Thus, the additional stabilization of the compositions and dosage forms may be needed.

Furthermore, it has been surprisingly found that the content of the N-nitrosamine impurity in a basic environment, when the sample is stressed, can increase significantly, while with the addition of a nitrite scavenger, which is ascorbic acid, the increase can be inhibited. However, Duloxetine, in particular Duloxetine hydrochloride, is susceptible to hydrolysis in an acidic environment, so there is a challenge of how to ensure the appropriate amount of nitrite scavenger, which is ascorbic acid, to limit the formation of the N-nitrosamine impurity, while maintaining the appropriate pH to ensure the hydrolytic stability of Duloxetine.

Moreover, it has been surprisingly found that a nitrite scavenger which is ascorbic acid, commonly known as vitamin C, is effective in reducing the formation of N-nitrosamines. It can act as a nitrite scavenger, meaning that it can react with nitrites to prevent them from forming N-nitrosamines. A nitrite scavenger which is ascorbic acid can be added as an excipient to block nitrosation reactions, thereby reducing the risk of N-nitrosamine formation. However, because of Duloxetine's sensitivity to acidic environment, the addition of an acidic nitrite scavenger which is ascorbic acid to a first coating layer containing the Duloxetine can be problematic, because hydrolysis of Duloxetine leads to impurity 1-naphthol. This impurity can cause a number of side effects, such as nausea and vomiting. Surprisingly, by adding a larger amount of acidic nitrite scavenger which is ascorbic acid to a second coating layer and leaving only a smaller amount of it in the first coating layer, a favorable effect on limiting the increase in the N-nitrosamine impurity can be achieved, while at the same time Duloxetine is not exposed to an environment that is too acidic. Thus, the N-nitrosamine levels of the final composition can be limited to safe levels, while chemical stability of the final pharmaceutical composition or dosage form can also be ensured.

In particular, it has been surprisingly found that advantageous compositions comprising Duloxetine can be provided, preferably as Duloxetine containing pellets, comprisingh two coating layers, preferably three or four coating layers. The first coating layer, which preferably is applied on an inert sugar pellet core, contains the active ingredient Duloxetine, preferably Duloxetine hydrochloride. Duloxetine is sensitive to acidic environment, which is why the purpose of the second coating layer preferably is to physically separate the active ingredient from the acidic gastro-resistant polymer, preferably found in the third coating layer. The thickness of the second coating layer can also affect the rate of release of the active ingredient from the compositions. The third coating layer preferably contains a gastroresistant polymer and is intended to protect the active ingredient from acidic conditions in the stomach. The fourth coating layer is preferably intended to achieve the appropriate appearance of the compositions. Further, advantageous dosage forms, preferably gastro-resistant capsules, comprising the compositions can be provided.

Still further, it has been surprisingly found that the composition according to the invention can be prepared in a process, which does not comprise organic solvents such as acetone. Thus, the process according to the invention is less environmentally burdensome.

Moreover, it has been surprisingly found that, when the active ingredient coating layer does not comprise a filler such as sucrose, agglomeration can be reduced from approximately 2 to 3% per batch to 0%.

A first aspect of the invention relates to a pharmaceutical composition comprising or essentially consisting of
- a core;
- an active ingredient coating layer comprising Duloxetine or a physiologically acceptable salt thereof, preferably Duloxetine hydrochloride;
- an intermediate coating layer;
- an enteric coating layer; and
- optionally, a finishing coating layer;
wherein the active ingredient coating layer and the intermediate coating layer comprises a nitrite scavenger, which is ascorbic acid.

The composition according to the invention preferably is a granular composition; preferably wherein the granules are pellets.

The composition according to the invention preferably is a multiparticulate composition.

For the purpose of the specification, *"pellets"* are granules with an almost spherical form.

Unless expressly stated otherwise, all percentages are expressed as weight percent. Unless expressly stated otherwise, all percentages are based on the total weight of the pharmaceutical composition according to the invention.

For the purpose of the specification, unless expressly stated otherwise, *"essentially consisting of"* or *"essentially the total amount"* means at least 95 wt.-%, preferably at least 98 wt.-%, more preferably at least 99 wt.-%.

Unless expressly stated otherwise, all references to Ph. Eur., ASTM, EN ISO and the like refer to the official version that is valid on January 1, 2024.

The term *"Duloxetine",* as used herein, refers to Duloxetine and pharmaceutically acceptable salts thereof, including hydrates and solvates thereof, and crystalline or amorphous forms thereof. The preferred form is the hydrochloride salt. Duloxetine used in the pharmaceutical composition form according to the invention may be prepared according to any manufacturing process known from the state of the art.

The term *"composition",* as used herein, preferably refers to a plurality of physically discrete units. Each discrete unit preferably comprises the core, the active ingredient coating layer comprising Duloxetine or a physiologically acceptable salt thereof, the intermediate coating layer, the enteric coating layer, and optionally, and the finishing coating layer, in form of a granule, preferably a pellet.

The pharmaceutical composition according to the invention comprises the nitrite scavenger in the active ingredient coating layer and the intermediate coating layer.

The nitrite scavenger is ascorbic acid.

In preferred embodiments, the total weight content of the nitrite scavenger is at most 3.0 wt.%, preferably at most 2.5 wt.%, more preferably at most 2.0 wt.%, still more preferably at most 1.5 wt.%, yet more preferably at most 1.0 wt.%, even more preferably at most 0.8 wt.%, most preferably at most 0.6 wt.%, and in particular at most 0.4 wt.-%, in each case relative to the total weight of the composition.

In preferred embodiments, the total weight content of the nitrite scavenger is at least 0.01 wt.%, preferably at least 0.05 wt.%, more preferably at least 0.1 wt.%, still more preferably at least 0.2 wt.%, yet more preferably at least 0.3 wt.%, even more preferably at least 0.4 wt.%, most preferably at least 0.6 wt.%, and in particular at least 0.7 wt.-%, in each case relative to the total weight of the composition.

In preferred embodiments, the total weight content of the nitrite scavenger is within the range of from 0.05 to 1.4 wt.-%, preferably 0.1 to 1.2 wt.-%, more preferably 0.2 to 1.0 wt.-%, still more preferably 0.4 to 0.8 wt.-%, yet more preferably 0.5 to 0.7 wt.-%, and most preferably 0.55 to 0.65 wt.-%, in each case relative to the total weight of the composition.

Preferably, the relative ratio of nitrite scavenger contained in the intermediate coating layer to nitrite scavenger contained in the active ingredient coating layer is within the range of from 5.0:1 to 29:1, preferably 7.0:1 to 27:1, more preferably 9.0:1 to 25:1, still more preferably 11:1 to 23:1, yet more preferably 13:1 to 21:1, and most preferably 15:1 to 19:1.

In preferred embodiments, at least 30 wt.-% of the nitrite scavenger contained in the composition is contained in the intermediate coating layer; preferably at least 35 wt.-%, more preferably at least 40 wt.-%, still more preferably at least 45 wt.%, yet more preferably at least 50 wt.%, even more preferably at least 55 wt.%, most preferably at least 60 wt.%, and in particular at least 65 wt.-%, in each case relative to the total weight of the composition.

In preferred embodiments, at least 70 wt.-% of the nitrite scavenger contained in the composition is contained in the intermediate coating layer; preferably at least 75 wt.-%, more preferably at least 80 wt.-%, still more preferably at least 83 wt.%, yet more preferably at least 86 wt.%, even more preferably at least 89 wt.%, most preferably at least 92 wt.%, and in particular at least 95 wt.-%, in each case relative to the total weight of the composition.

In preferred embodiments, at most 60 wt.-% of the nitrite scavenger contained in the composition is contained in the active ingredient coating layer; preferably at most 55 wt.%, more preferably at most 50 wt.%, still more preferably at most 45 wt.%, yet more preferably at most 40 wt.%, even more preferably at most 35 wt.%, most preferably at most 30 wt.%, and in particular at most 25 wt.-%, in each case relative to the total weight of the composition.

In preferred embodiments, at most 20 wt.-% of the nitrite scavenger contained in the composition is contained in the active ingredient coating layer; preferably at most 18 wt.%, more preferably at most 16 wt.%, still more preferably at most 14 wt.%, yet more preferably at most 12 wt.%, even more preferably at most 10 wt.%, most preferably at most 8.0 wt.%, and in particular at most 6.0 wt.-%, in each case relative to the total weight of the composition.

In preferred embodiments, the weight content of the nitrite scavenger contained in the active ingredient coating layer is at most 1.0 wt.%, preferably at most 0.6 wt.%, more preferably at most 0.4 wt.%, still more preferably at most 0.2 wt.%, yet more preferably at most 0.1 wt.%, even more preferably at most 0.08 wt.%, most preferably at most 0.06 wt.%, and in particular at most 0.04 wt.-%, in each case relative to the total weight of the composition.

In preferred embodiments, the weight content of the nitrite scavenger contained in the active ingredient coating layer is at least 0.001 wt.%, preferably at least 0.005 wt.%, more preferably at least 0.01 wt.%, still more preferably at least 0.02 wt.%, yet more preferably at least 0.04 wt.%, even more preferably at least 0.06 wt.%, most preferably at least 0.08 wt.%, and in particular at least 0.1 wt.-%, in each case relative to the total weight of the composition.

In preferred embodiments, the weight content of the nitrite scavenger contained in the active ingredient coating layer is within the range of from 0.005 to 0.2 wt.-%, preferably 0.008 to 0.1 wt.-%, still more preferably 0.01 to 0.07 wt.-%, and most preferably 0.03 to 0.05 wt.-%, in each case relative to the total weight of the composition.

In preferred embodiments, the weight content of the nitrite scavenger contained in the intermediate coating layer is at most 3.0 wt.%, preferably at most 2.5 wt.%, more preferably at most 2.0 wt.%, still more preferably at most 1.5 wt.%, yet more preferably at most 1.0 wt.%, even more preferably at most 0.8 wt.%, most preferably at most 0.6 wt.%, and in particular at most 0.4 wt.-%, in each case relative to the total weight of the composition.

In preferred embodiments, the weight content of the nitrite scavenger contained in the intermediate coating layer is at least 0.01 wt.%, preferably at least 0.05 wt.%, more preferably at least 0.1 wt.%, still more preferably at least 0.2 wt.%, yet more preferably at least 0.3 wt.%, even more preferably at least 0.4 wt.%, most preferably at least 0.5 wt.%, and in particular at least 0.6 wt.-%, in each case relative to the total weight of the composition.

In preferred embodiments, the weight content of the nitrite scavenger contained in the intermediate coating layer is within the range of from 0.05 to 1.5 wt.-%, preferably 0.1 to 1.0 wt.-%, still more preferably 0.2 to 0.8 wt.-%, and most preferably 0.4 to 0.6 wt.-%, in each case relative to the total weight of the composition.

Preferably a smaller amount of nitrite scavenger is contained in the active ingredient coating layer than in the intermediate coating layer. In this way, the active ingredient can be in contact with the nitrite scavenger, which is ascorbic acid, only through the interface between the layers, i.e. the influence of nitrite scavenger, which is ascorbic acid, on the pH in the vicinity of Duloxetine is smaller. It has been surprisingly found that the stability of the composition according to the invention under stressed conditions can be improved, when a the active ingredient coating layer comprises a smaller amount of nitrite scavenger compared to the intermediate coating layer.

Preferably, the pharmaceutical composition according to the invention has a content of N-nitrosamine impurity, preferably N-nitroso-Duloxetine, of at most 0.5 ppm, preferably at most 0.4 ppm, more preferably at most 0.3 ppm, still more preferably at most 0.2 ppm, yet more preferably at most 0.1 ppm, even more preferably at most 0.08 ppm, most preferably at most 0.06 ppm, and in particular at most 0.04 ppm, in each case by weight (ppmw); preferably after preparation of the composition.

Preferably, the pharmaceutical composition according to the invention after storage under accelerated storage conditions (30 °C/65 %RH) for 6 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Preferably, the pharmaceutical composition according to the invention after storage under accelerated storage conditions (40 °C/75 %RH) for 3 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Preferably, the pharmaceutical composition according to the invention after storage under accelerated storage conditions (40 °C/75 %RH) for 6 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Preferably, the pharmaceutical composition according to the invention after storage under accelerated storage conditions (50 °C/75 %RH) for 1 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Preferably, the Duloxetine or the physiologically acceptable salt thereof is present as Duloxetine hydrochloride.

In preferred embodiments, the total weight content of the Duloxetine or the physiologically acceptable salt thereof is at most 50 wt.%, preferably at most 45 wt.%, more preferably at most 40 wt.%, still more preferably at most 35 wt.%, yet more preferably at most 30 wt.%, even more preferably at most 28 wt.%, most preferably at most 26 wt.%, and in particular at most 24 wt.-%, in each case based upon an equivalent weight of the non-salt form of Duloxetine and in each case relative to the total weight of the composition.

In preferred embodiments, the total weight content of the Duloxetine or the physiologically acceptable salt thereof is at least 7.5 wt.%, preferably at least 10 wt.%, more preferably at least 12.5 wt.-%, still more preferably at least 15 wt.%, yet more preferably at least 17.5 wt.%, even more preferably at least 20 wt.%, most preferably at least 22.5 wt.%, and in particular at least 25 wt.-%, in each case based upon an equivalent weight of the non-salt form of Duloxetine and in each case relative to the total weight of the composition.

In preferred embodiments, the total weight content of the Duloxetine or the physiologically acceptable salt thereof is within the range of from 10 to 40 wt.-%, preferably 15 to 35 wt.-%, still more preferably 20 to 30 wt.-%, and most preferably 22 to 28 wt.-%, in each case based upon an equivalent weight of the non-salt form of Duloxetine and in each case relative to the total weight of the composition.

In preferred embodiments, essentially the total amount of the Duloxetine or the physiologically acceptable salt thereof contained in the composition is contained in the active ingredient coating layer.

Preferably, the Duloxetine or the physiologically acceptable salt thereof is the sole pharmacologically active ingredient contained in the composition.

The composition according to the invention comprises a core. Preferably, the core is an inert core; preferably an inert bead.

Preferably, the core comprises or essentially consists of sucrose, starch, microcrystalline cellulose, vegetable gums, waxes, and mixtures thereof; preferably sucrose; more preferably sucrose pellets or spheres.

Preferably, the composition according to the invention comprises one or more binders, one or more fillers, one or more lubricants, one or more gastroresistant (enteric) polymers, one or more plastifying agents, and/or mixtures thereof.

Preferably, the composition according to the invention comprises one or more binders.

Preferably, the one or more binders are selected from the group consisting of cellulose ethers, preferably selected from hydroxyethyl cellulose, hydroxypropyl cellulose, and hydroxypropyl methyl cellulose; gelatin; polyvinylpyrrolidone; and mixtures thereof; preferably hydroxypropyl methyl cellulose (hypromellose/HPMC).

Preferably, the one or more binders are contained in the active ingredient coating layer and/or the intermediate coating layer and/or the finishing coating layer.

In preferred embodiments, the total weight content of the one or more binders is within the range of from 4.0 to 24 wt.-%, preferably 6.0 to 22 wt.-%, still more preferably 8.0 to 20 wt.-%, and most preferably 10 to 18 wt.-%; in each case relative to the total weight of the composition.

Preferably, the composition according to the invention comprises one or more fillers.

Preferably, the one or more fillers are selected from the group consisting of mono- or oligosaccharides or their derivatives; preferably selected from lactose, e.g. lactose monohydrate, anhydrous lactose, spray dried and/or granulated lactose; sucrose, fructose, dextrates, saccharose, raffinose, trehalose, fructose, and dextrin; sugar alcohols; preferably selected from xylitol, mannitol, maltitol, isomalt, and sorbitol; cellulose or cellulose derivatives; preferably powdered cellulose, microcrystalline cellulose, or silicified microcrystalline cellulose; starch or starch derivatives; preferably low moisture starch, corn starch, pregelatinized starch, or low moisture pregelatinized starch; salts of phosphoric acid; preferably calcium salts of phosphoric acid; preferably selected from calcium hydrogen phosphate anhydrous and calcium hydrogen phosphate hydrate; salts of carbonic acid; preferably calcium carbonate, sodium carbonate, potassium carbonate, calcium hydrogencarbonate, sodium hydrogencarbonate, or potassium hydrogencarbonate; salts of lactic acid; preferably calcium lactate; and mixtures thereof; preferably mono- or oligosaccharides; more preferably sucrose.

Preferably, the one or more fillers are contained in the active ingredient coating layer and/or the intermediate coating layer; preferably the intermediate coating layer.

In preferred embodiments, the total weight content of the one or more fillers is within the range of from 0.5 to 20 wt.-%, preferably 1.0 to 15 wt.-%, still more preferably 2.0 to 10 wt.-%, and most preferably 4.0 to 8.0 wt.-%; in each case relative to the total weight of the composition.

Preferably, the composition according to the invention comprises one or more lubricants.

For the purpose of the specification a lubricant is considered equivalent to a glidant. Thus, both excipients perform the same functions, namely to help to reduce friction between surfaces in mutual contact.

Preferably, the one or more lubricants are selected from the group consisting of colloidal silica, talc, magnesium trisilicate, and mixtures thereof; preferably talc.

Preferably, the one or more lubricants are contained in the intermediate coating layer and/or the enteric coating layer.

In preferred embodiments, the total weight content of the one or more lubricants is within the range of from 4.0 to 24 wt.-%, preferably 6.0 to 22 wt.-%, still more preferably 8.0 to 20 wt.-%, and most preferably 10 to 18 wt.-%; in each case relative to the total weight of the composition.

Preferably, the composition according to the invention comprises one or more gastroresistant polymers (enteric polymers).

Preferably, the one or more gastroresistant polymers are selected from the group consisting of methacrylic acid copolymers, cellulose acetate phthalate, cellulose acetate succinate, polymethacrylic acid, hydroxypropyl methylcellulose phthalate, hydroxypropyl methylcellulose acetate succinate, polyvinyl acetate phthalate, hydroxyethyl ethyl cellulose phthalate, cellulose acetate tetrahydrophthalate, and mixtures thereof; preferably hydroxypropyl methylcellulose phthalate or hydroxypropyl methylcellulose acetate succinate; more preferably hydroxypropyl methylcellulose acetate succinate.

Preferably, the one or more gastroresistant polymers are contained in the enteric coating layer.

In preferred embodiments, the total weight content of the one or more gastroresistant polymers is within the range of from 6.0 to 24 wt.-%, preferably 8.0 to 22 wt.-%, still more preferably 10 to 20 wt.-%, and most preferably 15 to 18 wt.-%; in each case relative to the total weight of the composition.

Preferably, the composition according to the invention comprises one or more plastifying agents (plasticizers).

Preferably, the one or more plastifying agents are selected from the group consisting of propylene glycol, triethyl citrate, tributyl citrate, dibutyl sebacate, acetyl tributyl citrate, glyceryl monostearate, triacetin, polyethylene glycol, diethyl phthalate, acetylated monoglycerides, diacetylated monoglycerides, cetyl alcohol, and mixtures thereof; preferably triethyl citrate.

Preferably, the one or more plastifying agents are contained in the enteric coating layer.

In preferred embodiments, the total weight content of the one or more gastroresistant polymers is within the range of from 0.5 to 6.0 wt.-%, preferably 1.0 to 5.0 wt.-%, still more preferably 1.5 to 4.5 wt.-%, and most preferably 2.0 to 4.0 wt.-%; in each case relative to the total weight of the composition.

Preferably, the composition according to the invention comprises
- the core;
- the active ingredient coating layer comprising Duloxetine or a physiologically acceptable salt thereof, preferably Duloxetine hydrochloride;
- the intermediate coating layer comprising the nitrite scavenger with a weight content within the range of from 0.2 to 0.8 wt.-%, relative to the total weight of the composition;
- the enteric coating layer; and
- optionally, the finishing coating layer.

Preferably, the composition according to the invention comprises
- the core;
- the active ingredient coating layer comprising Duloxetine or a physiologically acceptable salt thereof, preferably Duloxetine hydrochloride, and comprising the nitrite scavenger with a weight content within the range of from 0.01 to 0.07 wt.-%, relative to the total weight of the composition;
- the intermediate coating layer;
- the enteric coating layer; and
- optionally, the finishing coating layer.

Preferably, the composition according to the invention comprises
- the core;
- the active ingredient coating layer comprising Duloxetine or a physiologically acceptable salt thereof, preferably Duloxetine hydrochloride, and comprising the nitrite scavenger;
- the intermediate coating layer comprising the nitrite scavenger;
- the enteric coating layer; and
- optionally, the finishing coating layer;
wherein the total weight content of the nitrite scavenger is within the range of from 0.5 to 0.7 wt.-%, relative to the total weight of the composition.

Preferably, the composition according to the invention comprises
- the core;
- the active ingredient coating layer comprising Duloxetine or a physiologically acceptable salt thereof, preferably Duloxetine hydrochloride, and comprising the nitrite scavenger;
- the intermediate coating layer, comprising the nitrite scavenger;
- the enteric coating layer; and
- optionally, the finishing coating layer;
wherein the relative ratio of nitrite scavenger contained in the intermediate coating layer to nitrite scavenger contained in the active ingredient coating layer is within the range of from 5.0:1 to 29: 1.

Preferably, the active ingredient coating layer is positioned between the core and the intermediate coating layer; preferably in direct contact with the core and/or the intermediate coating layer.

Preferably, the intermediate coating layer is positioned between the active ingredient coating layer and the enteric coating layer; preferably in direct contact with the active ingredient coating layer and/or the enteric coating layer.

Preferably, the enteric coating layer is positioned between the intermediate coating layer and the finishing coating layer; preferably in direct contact with the intermediate coating layer and/or the finishing coating layer.

Another aspect of the invention relates to a pharmaceutical dosage form comprising the pharmaceutical composition according to the invention. This aspect and its preferred embodiments according to paragraphs [0092] to [0099] are not part of the claims.

The dosage form according to the invention is preferably for oral administration.

Preferably, the dosage form according to the invention is selected from the group consisting of tablets, minitablets, microtablets, coated tablets, coated minitablets, coated microtablets, pills, powders, lozenges, sachets, capsules, and suppositories; preferably capsules; more preferably gelatin capsules.

In preferred embodiments, the weight content of the dose of the Duloxetine or the physiologically acceptable salt thereof is within the range of from 1 to 200 mg, preferably 20 to 180 mg, still more preferably 40 to 150 mg, most preferably 60 mg or 90 mg.

Preferably, the pharmaceutical dosage form according to the invention has a content of N-nitrosamine impurity, preferably N-nitroso-Duloxetine, of at most 0.5 ppm, preferably at most 0.4 ppm, more preferably at most 0.3 ppm, still more preferably at most 0.2 ppm, yet more preferably at most 0.1 ppm, even more preferably at most 0.08 ppm, most preferably at most 0.06 ppm, and in particular at most 0.04 ppm, in each case by weight (ppmw); preferably after preparation of the composition.

Preferably, the pharmaceutical dosage form according to the invention after storage under accelerated storage conditions (30 °C/65 %RH) for 6 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Preferably, the pharmaceutical dosage form according to the invention after storage under accelerated storage conditions (40 °C/75 %RH) for 3 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Preferably, the pharmaceutical dosage form according to the invention after storage under accelerated storage conditions (40 °C/75 %RH) for 6 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Preferably, the pharmaceutical dosage form according to the invention after storage under accelerated storage conditions (50 °C/75 %RH) for 1 months, has a content of N-nitrosamine impurity, preferably N-nitroso-Duloxetine, of at most 0.8 ppm, preferably at most 0.7 ppm, more preferably at most 0.6 ppm, still more preferably at most 0.5 ppm, yet more preferably at most 0.4 ppm, even more preferably at most 0.3 ppm, most preferably at most 0.2 ppm, and in particular at most 0.1 ppm, in each case by weight (ppmw).

Another aspect of the invention relates to a process for the preparation of a pharmaceutical composition according to the invention, comprising the steps of:
(a) preparing a dispersion of Duloxetine or a physiologically acceptable salt thereof, preferably comprising one or more binders, one or more fillers, and the nitrite scavenger;
(b) coating a core with the dispersion provided in step (a);
(c) preparing a dispersion comprising the nitrite scavenger, one or more binders, one or more fillers, and/or one or more lubricants;
(d) coating the granules obtained in step (b) with the dispersion provided in step (c);
(e) preparing an enteric dispersion comprising one or more gastroresistant polymers, one or more plastifying agents, and/or one or more lubricants; wherein the one or more gastroresistant polymers are preferably at least partially neutralized;
(f) coating the granules obtained in step (d) with the dispersion provided in step (e);
(g) optionally, preparing a finishing dispersion comprising one or more binders and/or one or more pigments; and
(h) optionally, coating the granules obtained in step (f) with the dispersion provided in step (g).

Preferably, in step (e) no acetone is used as dispersion solvent; preferably no organic solvent; more preferably water is used as dispersion solvent.

Another aspect of the invention relates to a pharmaceutical composition obtainable by the process according to the invention.

Another aspect of the invention relates to a process for the preparation of a pharmaceutical dosage form according to the invention, the process comprising the steps of:
(A) providing a pharmaceutical composition according to the invention;
(B) preparing an encapsulation mixture by adding one or more lubricants to the pharmaceutical composition provided in step (A); and
(C) filling the encapsulation mixture provided in step (B) into capsules; preferably gelatine capsules. This aspect is not part of the claims.

The following examples further illustrate the invention but are not to be construed as limiting its scope:

### Example 1: Not according to the claims.

Pharmaceutical compositions were prepared having the following composition:

| Ingredient (for 60 mg strength) | | Amount | |
|---|---|---|---|
| | | [mg] | [wt.-%] |
| SUGAR PELLETS (500-600 µm) | Pellets | 55.00 | 21.2 |

| *Active ingredient coating layer* | | | |
|---|---|---|---|
| DULOXETINE HYDROCHLORIDE | Active ingredient | 67.35 | 26.0 |
| HYPROMELLOSE (6CP) | binder | 8.00 | 3.1 |
| SUCROSE | filler | 16.00 | 6.2 |
| PURIFIED WATER | Dispersion medium | q.s. | |

| *Intermediate coating layer* | | | |
|---|---|---|---|
| HYPROMELLOSE (6CP) | binder | 14.00 | 5.4 |
| SUCROSE | Filler | 20.20 | 7.8 |
| TALC | Lubricant | 33.70 | 13.0 |
| PURIFIED WATER | Dispersion medium | q.s. | |

| *Enteric coating layer* | | | |
|---|---|---|---|
| HPMC PHTHALATE HP-50 | Gastroresistant polymer | 37.08 | 14.3 |
| TRIETHYL CITRATE | Plastifying agent | 3.71 | 1.4 |
| TALC | Lubricant | 4.08 | 1.6 |
| ACETONE | Dispersion medium | q.s. | |
| PURIFIED WATER | Dispersion medium | q.s. | |

The pharmaceutical compositions were prepared as follows:
a) Preparation of a dispersion for the active ingredient coating layer: Hypromellose and sucrose were dissolved in purified water. In the resulting clear solution Duloxetine hydrochloride was suspended.
b) Coating neutral pellets with the active ingredient coating layer: Sugar pellets were heated to 36 °C in the FBD pellet coater. The dispersion of step a) was sprayed onto the pellets from below. The temperature of the pellets was maintained at between 34 and 38 °C during spraying. After spraying the pellets were dried to a temperature of 45 °C. Any resulting dust (< 0.6 mm) and agglomerates (> 1.0 mm) were sieved off.
c) Preparation of a dispersion for the intermediate coating layer: Hypromellose and sucrose were dissolved in purified water. In the resulting clear solution talc was suspended.
d) Coating pellets with the intermediate coating layer: The pellets of step b) were heated to 36 °C in the FBD pellet coater. The dispersion of step c) was sprayed onto the pellets. The temperature of the pellets was maintained at between 34 and 38 °C during spraying. After spraying the pellets were dried to a temperature of 45 °C. Any resulting dust (< 0.6 mm) and agglomerates (> 1.25 mm) were sieved off.
e) Preparation of a dispersion for the enteric coating layer: HPMC phthalate and triethyl citrate were dissolved in acetone. Talc was suspended in purified water and added to the solution of HPMC phtalate and triethyl citrate.
f) Coating pellets with the enteric coating layer (gastroresistant coating): The pellets of step d) were heated to 24 °C in the FBD pellet coater and sprayed from below with the dispersion of step e). The temperature of the pellets was maintained at between 20 and 26 °C during spraying. After spraying the pellets were dried to a final temperature of 45 °C. Any resulting dust (< 0.6 mm) and agglomerates (> 1.4 mm) were sieved off.

### A) influence of nitrite content in lubricant:

Talc with different nitrite contents was used in the intermediate coating layer for three compositions. The content of N-nitrosamine impurity in the pellets was determined directly after preparation (t₀) and again after three and six months at 40 °C/75%RH, respectively. The different nitrite contents of the talc and the amount of N-nitrosamine impurity are summarized in the table here below:

| | nitrite content [ppm] | N-nitrosamine impurity [ppm] | | |
|---|---|---|---|---|
| | | t₀ | 40 °C/75%RH 3 months | 40 °C/75%RH 6 months |
| 1-1 | 0.31 | 0.12 | 0.71 | 0.85 |
| 1-2 | 1.13 | 0.11 | 0.73 | 0.89 |
| 1-3 | 1.65 | 0.12 | 0.69 | 0.83 |

As demonstrated, the nitrite content in the talc has no effect on the increase of N-nitrosamine impurities. Further, the safe limit of 0.83 ppm for N-nitroso-Duloxetine was already exceeded after 6 months at 40°C/75%RH.

### B) influence of nitrite content in capsules:

Gelatin capsules containing the pharmaceutical composition were prepared. Gelatin capsules with different nitrite contents were used. The content of N-nitrosamine impurity in the capsules containing the pharmaceutical composition was determined directly after preparation (t₀) and again after three and six months at 40 °C/75%RH as well as after six months at 30 °C/65%RH. The different nitrite contents of the gelatine capsules and the amount of N-nitrosamine impurity are summarized in the table here below:

| | nitrite content [ppm] | N-nitrosamine impurity [ppm] | | | |
|---|---|---|---|---|---|
| | | t₀ | 30 °C/65%RH 6 months | 40 °C/75%RH 3 months | 40 °C/75%RH 6 months |
| 1-4 | 0.18 | 0.62 | 1.4 | 1.5 | 1.5 |
| 1-5 | 0.52 | 0.78 | 1.8 | 1.9 | 2.0 |
| 1-6 | 1.4 | 0.69 | 2.0 | 2.3 | 2.6 |

As demonstrated, the content of N-nitrosamine impurity is increased after encapsulation, compared to pharmaceutical composition before encapsulation (Examples 1-1 to 1-3 vs Examples 1-4 to 1-6). Further, with increased nitrite content in the capsules, the amount of N-nitrosamine impurity after stressed conditions is also increased. Thus, the nitrite content in the gelatine capsules influences the increase of N-nitrosamine impurities. Further, the safe limit of 0.83 ppm for N-nitroso-Duloxetine was already exceeded after 3 months at 40°C/75%RH.

### Example 2 - influence of nitrite scavengers:

Pharmaceutical compositions were prepared having the following composition: Only the compositions where the active ingredient coating layer and the intermediate coating layer comprise ascorbic acid as a nitrite scavenger are according to the claims.

| | | Ex. 2-1 | | Ex. 2-2 | |
|---|---|---|---|---|---|
| Ingredient (for 60 mg strength) | | Amount | | Amount | |
| | | [mg] | [wt.-%] | [mg] | [wt.-%] |
| SUGAR PELLETS (500-600 µm) | Pellets | 55.00 | 21.2 | 55.00 | 21.2 |

| *Active ingredient coating layer* | | | | | |
|---|---|---|---|---|---|
| DULOXETINE HYDROCHLORIDE | Active ingredient | 67.35 | 26.0 | 67.35 | 26.0 |
| HYPROMELLOSE (6CP) | binder | 8.00 | 3.1 | 8.00 | 3.1 |
| SUCROSE | filler | 15.00 | 5.8 | 15.90 | 6.1 |
| SODIUM HYDROXIDE | Basic nitrite scavenger | 1.00 | 0.4 | | |
| ASCORBIC ACID | Acidic nitrite scavenger | | | 0.10 | 0.04 |
| PURIFIED WATER | Dispersion medium | q.s. | | q.s. | |

| *Intermediate coating layer* | | | | | |
|---|---|---|---|---|---|
| HYPROMELLOSE (6CP) | binder | 14.00 | 5.4 | 14.00 | 5.4 |
| SUCROSE | Filler | 20.20 | 7.8 | 20.20 | 7.8 |
| TALC | Lubricant | 33.70 | 13.0 | 33.70 | 13.0 |
| PURIFIED WATER | Dispersion medium | q.s. | | q.s. | |

| *Enteric coating layer* | | | | | |
|---|---|---|---|---|---|
| HPMC PHTHALATE HP-50 | Gastroresistant polymer | 37.08 | 14.3 | 37.08 | 14.3 |
| TRIETHYL CITRATE | Plastifying agent | 3.71 | 1.4 | 3.71 | 1.4 |
| TALC | Lubricant | 4.08 | 1.6 | 4.08 | 1.6 |
| ACETONE | Dispersion medium | q.s. | | q.s. | |
| PURIFIED WATER | Dispersion medium | q.s. | | q.s. | |

| | | Ex. 2-3 | | Ex. 2-4 | |
|---|---|---|---|---|---|
| Ingredient (for 60 mg strength) | | Amount | | Amount | |
| | | [mg] | [wt.-%] | [mg] | [wt.-%] |
| SUGAR PELLETS (500-600 µm) | Pellets | 55.00 | 21.2 | 55.00 | 21.2 |

| *Active ingredient coating layer* | | | | | |
|---|---|---|---|---|---|
| DULOXETINE HYDROCHLORIDE | Active ingredient | 67.35 | 26.0 | 67.35 | 26.0 |
| HYPROMELLOSE (6CP) | binder | 8.00 | 3.1 | 8.00 | 3.1 |
| SUCROSE | filler | 16.00 | 6.2 | 15.90 | 6.1 |
| SODIUM HYDROXIDE | Basic nitrite scavenger | | | | |
| ASCORBIC ACID | Acidic nitrite scavenger | | | 0.10 | 0.04 |
| PURIFIED WATER | Dispersion medium | q.s. | | q.s. | |

| *Intermediate coating layer* | | | | | |
|---|---|---|---|---|---|
| HYPROMELLOSE (6CP) | binder | 14.00 | 5.4 | 14.00 | 5.4 |
| SUCROSE | Filler | 20.20 | 7.8 | 20.20 | 7.8 |
| ASCORBIC ACID | Acidic nitrite scavenger | 1.00 | 0.4 | 1.00 | 0.4 |
| TALC | Lubricant | 32.70 | 12.6 | 32.70 | 12.6 |
| PURIFIED WATER | Dispersion medium | q.s. | | q.s. | |

| *Enteric coating layer* | | | | | |
|---|---|---|---|---|---|
| HPMC PHTHALATE HP-50 | Gastroresistant polymer | 37.08 | 14.3 | 37.08 | 14.3 |
| TRIETHYL CITRATE | Plastifying agent | 3.71 | 1.4 | 3.71 | 1.4 |
| TALC | Lubricant | 4.08 | 1.6 | 4.08 | 1.6 |
| ACETONE | Dispersion medium | q.s. | | q.s. | |
| PURIFIED WATER | Dispersion medium | q.s. | | q.s. | |

The pharmaceutical compositions were prepared as follows:
a) Preparation of a dispersion for the active ingredient coating layer: Hypromellose, optionally nitrite scavenger (sodium hydroxide or ascorbic acid) and sucrose were dissolved in purified water. In the resulting clear solution Duloxetine hydrochloride was suspended.
b) Coating neutral pellets with the active ingredient coating layer: Sugar pellets were heated to 36 °C in the FBD pellet coater. The dispersion of step a) was sprayed onto the pellets from below. The temperature of the pellets was maintained at between 34 and 38 °C during spraying. After spraying the pellets were dried to a temperature of 45 °C. Any resulting dust (< 0.6 mm) and agglomerates (> 1.0 mm) were sieved off.
c) Preparation of a dispersion for the intermediate coating layer: Hypromellose, sucrose and optionally ascorbic acid were dissolved in purified water. In the resulting clear solution talc was suspended.
d) Coating pellets with the intermediate coating layer: The pellets of step b) were heated to 36 °C in the FBD pellet coater. The dispersion of step c) was sprayed onto the pellets. The temperature of the pellets was maintained at between 34 and 38 °C during spraying. After spraying the pellets were dried to a temperature of 45 °C. Any resulting dust (< 0.6 mm) and agglomerates (> 1.25 mm) were sieved off.
e) Preparation of a dispersion for the enteric coating layer: HPMC phthalate and triethyl citrate were dissolved in acetone. Talc was suspended in purified water and added to the solution of HPMC phtalate and triethyl citrate .
f) Coating pellets with the enteric coating layer (gastroresistant coating): The pellets of step d) were heated to 24 °C in the FBD pellet coater and sprayed from below with the dispersion of step e). The temperature of the pellets was maintained at between 20 and 26 °C during spraying. After spraying the pellets were dried for at least 30 minutes to a final temperature of 45 °C. Any resulting dust (< 0.6 mm) and agglomerates (> 1.4 mm) were sieved off.

Gelatin capsules containing the pharmaceutical composition were prepared. The content of N-nitrosamine impurity in the capsules containing the pharmaceutical composition as described above was determined directly after preparation (t₀) and again after three and six months at 40 °C/75%RH as well as after six months at 30 °C/65%RH. The different nitrite scavenger and the amount of N-nitrosamine impurity are summarized in the table here below:

| | nitrite scavenger / layer | N-nitrosamine impurity [ppm] | | | |
|---|---|---|---|---|---|
| | | t₀ | 30 °C/65%RH 6 months | 40 °C/75%RH 3 months | 40 °C/75%RH 6 months |
| 2-1 | NaOH / first | 0.07 | 1.30 | 1.90 | 7.80 |
| 2-2 | ascorbic acid / first | 0.07 | 0.32 | 0.35 | 0.50 |
| 2-3 | ascorbic acid / second | 0.21 | 0.35 | 0.24 | 0.38 |
| 2-4 | ascorbic acid / first and second | 0.04 | 0.10 | 0.09 | 0.11 |

As demonstrated by Example 2-1, the content of the N-nitrosamine impurity in a basic environment after stressed conditions increases significantly, while with the addition of a nitrite scavenger such as ascorbic acid, the increase is inhibited. As further demonstrated by Examples 2-2 and 2-3, the addition of ascorbic acid to the active ingredient coating layer effectively inhibits the formation of N-nitrosamine impurities during the manufacturing process (Example 2-2), while a larger amount in the intermediate coating layer ensures long-term stability of the composition under stressed conditions (Example 2-3). The composition of Example 2-4, which included ascorbic acid in the first and in the second layer, achieved a low content of N-nitrosamine impurities at the initial point and a minimal increase at stressed conditions. Therefore, the appropriate amount of nitrite scavenger such as ascorbic acid ensures limiting the formation of the N-nitrosamine impurity, while maintaining the appropriate pH to ensure the hydrolytic stability of Duloxetine.

### Example 3 - influence of content of nitrite scavengers:

Pharmaceutical compositions were prepared having the following composition:

| | | Ex. 3-1 | | Ex. 3-2 | | Ex. 3-3 | | Ex. 3-4 | | Ex. 3-5 | | Ex. 3-6 | |
|---|---|---|---|---|---|---|---|---|---|---|---|---|---|
| Ingredient (for 60 mg strength) | | Amount | | Amount | | Amount | | Amount | | Amount | | Amount | |
| | | [mg] | [wt.-%] | [mg] | [wt.-%] | [mg] | [wt.-%] | [mg] | [wt.-%] | [mg] | [wt.-%] | [mg] | [wt.-%] |
| SUGAR PELLETS (500-600 µm) | Pellets | 55.00 | 19.4 | 55.00 | 19.4 | 55.00 | 19.5 | 55.00 | 19.4 | 55.00 | 21.1 | 55.00 | 18.3 |
| *Active ingredient coating layer* | | | | | | | | | | | | | |
| DULOXETINE HYDROCHLORIDE | Active ingredient | 67.35 | 23.7 | 67.35 | 23.7 | 67.35 | 23.8 | 67.35 | 23.8 | 67.35 | 25.4 | 67.35 | 22.4 |
| HYPROMELLOSE (6CP) | binder | 10.00 | 3.5 | 10.00 | 3.5 | 10.00 | 3.5 | 10.00 | 3.5 | 10.00 | 3.8 | 10.00 | 3.3 |
| ASCORBIC ACID | Acidic nitrite scavenger | 0.10 | 0.04 | 0.30 | 0.1 | 0.05 | 0.02 | 0.10 | 0.04 | 0.10 | 0.04 | 0.10 | 0.03 |
| PURIFIED WATER | Dispersion medium | q.s. | | q.s. | | q.s. | | q.s. | | q.s. | | q.s. | |
| *Intermediate coating layer* | | | | | | | | | | | | | |
| HYPROMELLOSE (6CP) | binder | 11.90 | 4.2 | 11.90 | 4.2 | 11.90 | 4.2 | 11.90 | 4.2 | 7.10 | 2.7 | 7.10 | 4.0 |
| SUCROSE | Filler | 17.20 | 6.1 | 17.20 | 6.1 | 17.20 | 6.1 | 17.20 | 6.1 | 10.30 | 3.9 | 10.30 | 5.7 |
| ASCORBIC ACID | Acidic nitrite scavenger | 1.70 | 0.6 | 1.70 | 0.6 | 0.40 | 0.1 | 0.85 | 0.3 | 1.70 | 0.7 | 1.70 | 0.6 |
| TALC | Lubricant | 27.80 | 9.8 | 27.80 | 9.8 | 27.80 | 9.8 | 27.80 | 9.8 | 16.70 | 6.4 | 16.70 | 9.2 |
| PURIFIED WATER | Dispersion medium | q.s. | | q.s. | | q.s. | | q.s. | | q.s. | | q.s. | |
| *Enteric coating layer* | | | | | | | | | | | | | |
| HYPROMELLOSE ACETATE SUCCINATE MF | Gastroresistant polymer | 44.60 | 15.7 | 44.60 | 15.7 | 44.60 | 15.8 | 44.60 | 15.8 | 44.60 | 17.1 | 55.75 | 18.5 |
| TRIETHYL CITRATE | Plastifying agent | 8.90 | 3.1 | 8.90 | 3.1 | 8.90 | 3.1 | 8.90 | 3.1 | 8.90 | 3.4 | 11.15 | 3.7 |
| TALC | Lubricant | 13.40 | 4.7 | 13.40 | 4.7 | 13.40 | 4.7 | 13.40 | 4.7 | 13.40 | 5.1 | 16.75 | 5.6 |
| AMMONIA 25% solution | Neutralizing agent | 2.15 | 0.8 | 2.15 | 0.8 | 2.15 | 0.8 | 2.15 | 0.8 | 2.15 | 0.8 | 1.03 | 0.4 |
| PURIFIED WATER | Dispersion medium | q.s. | | q.s. | | q.s. | | q.s. | | q.s. | | q.s. | |
| *Finishing coating layer* | | | | | | | | | | | | | |
| HYPROMELLOSE (6CP) | Binder | 17.00 | 6.0 | 17.00 | 6.0 | 17.00 | 6.0 | 17.00 | 6.0 | 17.00 | 6.0 | 17.00 | 5.7 |
| TITANIUM DIOXIDE | Pigment | 9.00 | 3.2 | 9.00 | 3.2 | 9.00 | 3.2 | 9.00 | 3.2 | 9.00 | 3.2 | 9.00 | 3.0 |
| PURIFIED WATER | Dispersion medium | q.s. | | q.s. | | q.s. | | q.s. | | q.s. | | q.s. | |

| | | Ex. 3-7 | | Ex. 3-8 | | Ex. 3-9 | |
|---|---|---|---|---|---|---|---|
| Ingredient (for 90 mg strength) | | Amount | | Amount | | Amount | |
| | | [mg] | [wt.-%] | [mg] | [wt.-%] | [mg] | [wt.-%] |
| SUGAR PELLETS (500-600 µm) | Pellets | 82.50 | 15.8 | 82.50 | 21.2 | 82.50 | 18.3 |

| *Active ingredient coating layer* | | | | | | | |
|---|---|---|---|---|---|---|---|
| DULOXETINE HYDROCHLORIDE | Active ingredient | 101.03 | 19.3 | 101.03 | 25.4 | 101.03 | 22.4 |
| HYPROMELLOSE (6CP) | binder | 15.00 | 2.9 | 15.00 | 3.8 | 15.00 | 3.3 |
| ASCORBIC ACID | Acidic nitrite scavenger | 0.15 | 0.03 | 0.15 | 0.04 | 0.15 | 0.03 |
| PURIFIED WATER | Dispersion medium | q.s. | | q.s. | | q.s. | |

| *Intermediate coating layer* | | | | | | | |
|---|---|---|---|---|---|---|---|
| HYPROMELLOSE (6CP) | binder | 17.85 | 3.4 | 10.65 | 2.7 | 10.65 | 4.0 |
| SUCROSE | Filler | 25.80 | 4.9 | 15.45 | 3.9 | 15.45 | 5.7 |
| ASCORBIC ACID | Acidic nitrite scavenger | 2.55 | 0.5 | 2.55 | 0.7 | 2.55 | 0.6 |
| TALC | Lubricant | 41.70 | 8.0 | 25.05 | 6.4 | 25.05 | 9.2 |
| PURIFIED WATER | Dispersion medium | q.s. | | q.s. | | q.s. | |

| *Enteric coating layer* | | | | | | | |
|---|---|---|---|---|---|---|---|
| HYPROMELLOSE ACETATE SUCCINATE MF | Gastroresistant polymer | 66.90 | 12.8 | 66.90 | 17.1 | 83.63 | 18.5 |
| TRIETHYL CITRATE | Plastifying agent | 13.35 | 2.6 | 13.35 | 3.4 | 16.73 | 3.7 |
| TALC | Lubricant | 20.10 | 3.8 | 20.10 | 5.1 | 25.13 | 5.6 |
| AMMONIA 25 solution | Neutralizing agent | 3.23 | 0.6 | 3.23 | 0.8 | 1.55 | 0.4 |
| PURIFIED WATER | Dispersion medium | q.s. | | q.s. | | q.s. | |

| *Finishing coating layer* | | | | | | | |
|---|---|---|---|---|---|---|---|
| HYPROMELLOSE (6CP) | Binder | 25.50 | 4.9 | 25.50 | 6.0 | 25.50 | 5.7 |
| TITANIUM DIOXIDE | Pigment | 13.50 | 2.6 | 13.50 | 3.2 | 13.50 | 3.0 |
| PURIFIED WATER | Dispersion medium | q.s. | | q.s. | | q.s. | |
| TALC | Glidant | 1.17 | 0.2 | | | | |
| GELATINE CAPSULES | Capsule | 96.00 | 18.4 | | | | |

The pharmaceutical compositions were prepared as follows:
a) Preparation of a dispersion for the active ingredient coating layer: Hypromellose and ascorbic acid were dissolved in purified water. In the resulting clear solution Duloxetine hydrochloride was suspended.
b) Coating neutral pellets with the active ingredient coating layer: Sugar pellets were heated to 36 °C in the FBD pellet coater. The dispersion of step a) was sprayed onto the pellets from below. The temperature of the pellets was maintained at between 34 and 38 °C during spraying. After spraying the pellets were dried to a temperature of 45 °C. Any resulting dust (< 0.6 mm) and agglomerates (> 1.0 mm) were sieved off.
c) Preparation of a dispersion for the intermediate coating layer: Hypromellose, sucrose and ascorbic acid were dissolved in purified water. In the resulting clear solution talc was suspended.
d) Coating pellets with the intermediate coating layer: The pellets of step b) were heated to 36 °C in the FBD pellet coater. The dispersion of step c) was sprayed onto the pellets. The temperature of the pellets was maintained at between 34 and 38 °C during spraying. After spraying the pellets were dried to a temperature of 45 °C. Any resulting dust (< 0.6 mm) and agglomerates (> 1.25 mm) were sieved off.
e) Preparation of a dispersion for the enteric coating layer: Hypromellose acetate succinate and talc were suspended in purified water and triethyl citrate was dissolved in the resulting suspension. Ammonia was added to the suspension, thereby achieving neutralization of the gastroresistant polymer and its partial dissolution.
f) Coating pellets with the enteric coating layer (gastroresistant coating): The pellets of step d) were heated to 33 °C in the FBD pellet coater and sprayed from below with the dispersion of step e). The temperature of the pellets was maintained at between 32 and 36 °C during spraying. After spraying the pellets were dried for at least 30 minutes to a final temperature of 45 °C. Any resulting dust (< 0.6 mm) and agglomerates (> 1.4 mm) were sieved off.
g) Preparation of a dispersion for the finishing coating layer: Hypromellose was dissolved in purified water. In the resulting clear solution titanium dioxide was suspended.
h) Coating the pellets with the finishing coating layer (outer coating): The pellets of step f) were heated to 33 °C in the FBD pellet coating machine. The dispersion of step g) was sprayed on the pellets from below. The temperature of the pellets was maintained at between 32 and 36 °C during spraying. After spraying the pellets were dried to a final product temperature of 45 °C. Any resulting dust (< 0.6 mm) and agglomerates (> 1.4 mm) were sieved off.

The pharmaceutical dosage forms were prepared as follows:
i) Preparation of the encapsulation mixture: Talc was added to the pellets of step h). The pellets were then mixed in a container mixer for at least 60 revolutions.
j) Encapsulation: The prescribed mass of the encapsulation mixture was filled into capsules using a capsule filling machine at a capsule filling speed of 50 - 140 cycles/minute.

The content of N-nitrosamine impurity in the capsules containing the pharmaceutical composition as described above was determined directly after preparation (t₀) and again after storage under stressed conditions. The amount of N-nitrosamine impurity is summarized in the table here below:

| | N-nitrosamine impurity [ppm] | | | | | | |
|---|---|---|---|---|---|---|---|
| | t₀ | 30°C /65%RH 6 months | 40°C /75%RH 1 month | 40°C /75%RH 2 month | 40 °C/75%RH 3 months | 40 °C/75%RH 6 months | 50 °C/75%RH 1 month |
| 3-2 | | | | | 0.11 | 0.12 | 0.12 |
| 3-3 | | | 0.13 | | 0.21 | 0.24 | 0.18 |
| 3-4 | 0.06 | 0.14 | 0.10 | 0.10 | 0.16 | 0.18 | 0.13 |
| 3-7 | 0.06 | 0.21 | 0.09 | 0.19 | 0.22 | 0.26 | 0.13 |

As demonstrated, ascorbic acid in the active ingredient coating layer and the intermediate coating layer effectively slows down the formation of the N-nitroso-Duloxetine impurity under stressed conditions, as all tested concentrations result in impurity levels below the safe limit of 0.83 ppm.

### General LC-MS method for determining the content of N-nitroso Duloxetine:

To determine the content of N-nitroso Duloxetine impurity in the final product, an LC-MS method was developed (DL 0.025 ppm, QL 0.083 ppm, linear range 0.083 ppm to 8 ppm).

The content of N-nitroso Duloxetine was analyzed using the following LC-MS method:
Preparation of mobile Phase A: 0.63 g of ammonium formate was weighed into a suitable beaker and dissolved in 1000 mL of purified (Milli-Q^{®}) water. 1 mL of formic acid was added to the solution while stirring. The solution was then filtered through 0.45 µm membrane filter and degased.

Preparation of mobile Phase B: 1 mL of formic acid ws added into 1000 mL of acetonitrile. The solution waas filtered through 0.45 µm membrane filter and degassed.

### Chromatographic conditions:

| | |
|---|---|
| HPLC Column | C18 column 100 × 3.0; 1.8 µm |
| Detections | MS (MRM, 344>123 and 344>183) |
| Flow rate | 0.8 mL/min |
| Injection volume | 5 µL |
| Column temperature | 40 °C |
| Run time | About 20 minutes |
| Diluent | Acetonitrile : water = 1 : 1 |

| Time (min) | Mobile Phase-A | Mobile Phase-B |
|---|---|---|
| 0 | 50 | 50 |
| 8.5 | 50 | 50 |
| 9 | 10 | 90 |
| 15 | 10 | 90 |
| 15.1 | 50 | 50 |
| 20 | 50 | 50 |

## Claims

1. A pharmaceutical composition comprising or essentially consisting of:
- a core;
- an active ingredient coating layer comprising Duloxetine or a physiologically acceptable salt thereof, preferably Duloxetine hydrochloride;
- an intermediate coating layer;
- an enteric coating layer; and
- optionally, a finishing coating layer;
wherein the active ingredient coating layer and the intermediate coating layer comprise ascorbic acid as a nitrite scavenger.

2. The composition according to claim 1, which is a granular composition; preferably pellets.

3. The composition according to any of the preceding claims, wherein the total weight content of ascorbic acid is within the range of from 0.05 to 1.4 wt.-%, preferably 0.1 to 1.2 wt.-%, more preferably 0.2 to 1.0 wt.-%, still more preferably 0.4 to 0.8 wt.-%, yet more preferably 0.5 to 0.7 wt.-%, and most preferably 0.55 to 0.65 wt.-%, in each case relative to the total weight of the composition.

4. The composition according to any of the preceding claims, wherein the relative ratio of ascorbic acid contained in the intermediate coating layer to ascorbic acid contained in the active ingredient coating layer is within the range of from 5.0:1 to 29:1, preferably 7.0:1 to 27:1, more preferably 9.0:1 to 25:1, still more preferably 11:1 to 23:1, yet more preferably 13:1 to 21:1, and most preferably 15:1 to 19:1.

5. The composition according to any of the preceding claims, which has a content of N-nitrosamine impurity, preferably N-nitroso-Duloxetine, of at most 0.5 ppm, preferably at most 0.4 ppm, more preferably at most 0.3 ppm, still more preferably at most 0.2 ppm, yet more preferably at most 0.1 ppm, even more preferably at most 0.08 ppm, most preferably at most 0.06 ppm, and in particular at most 0.04 ppm, in each case by weight (ppmw).

6. The composition according to any of the preceding claims, wherein the core comprises or essentially consists of sucrose, starch, microcrystalline cellulose, vegetable gums, waxes, and mixtures thereof; preferably sucrose; more preferably sucrose pellets or spheres.

7. The composition according to any of the preceding claims, which comprises one or more binders, one or more fillers, one or more lubricants, one or more gastroresistant polymers, one or more plastifying agents, and/or mixtures thereof.

8. The composition according to any of the preceding claims, which comprises one or more gastroresistant polymers; preferably selected from the group consisting of methacrylic acid copolymers, cellulose acetate phthalate, cellulose acetate succinate, polymethacrylic acid, hydroxypropyl methylcellulose phthalate, hydroxypropyl methylcellulose acetate succinate, polyvinyl acetate phthalate, hydroxyethyl ethyl cellulose phthalate, cellulose acetate tetrahydrophthalate, and mixtures thereof; preferably hydroxypropyl methylcellulose phthalate or hydroxypropyl methylcellulose acetate succinate; more preferably hydroxypropyl methylcellulose acetate succinate.

9. A process for the preparation of a pharmaceutical composition according to any of the preceding claims, comprising the steps of:
(a) preparing a dispersion of Duloxetine or a physiologically acceptable salt thereof, preferably comprising one or more binders, one or more fillers, and ascorbic acid;
(b) coating a core with the dispersion provided in step (a);
(c) preparing a dispersion comprising ascorbic acid, one or more binders, one or more fillers, and/or one or more lubricants;
(d) coating the pellets obtained in step (b) with the dispersion provided in step (c);
(e) preparing an enteric dispersion comprising one or more gastroresistant polymers, one or more plastifying agents, and/or one or more lubricants; wherein the one or more gastroresistant polymers are preferably at least partially neutralized;
(f) coating the pellets obtained in step (d) with the dispersion provided in step (e);
(g) optionally, preparing a finishing dispersion comprising one or more binders and/or one or more pigments; and
(h) optionally, coating the pellets obtained in step (f) with the dispersion provided in step (g).

10. The process according to claim 9, wherein in step (e) no acetone is used as dispersion solvent; preferably no organic solvent; more preferably water is used as dispersion solvent.

## Patentansprüche

1. Eine pharmazeutische Zusammensetzung, die Folgendes umfasst oder im Wesentlichen daraus besteht:
- einen Kern;
- eine Wirkstoffbeschichtungsschicht, die Duloxetin oder ein physiologisch verträgliches Salz davon, vorzugsweise Duloxetinhydrochlorid, enthält;
- eine Zwischenbeschichtungsschicht;
- eine magensaftresistente Beschichtungsschicht; und
- optional eine Abschlussbeschichtungsschicht;
wobei die Wirkstoffbeschichtungsschicht und die Zwischenbeschichtungsschicht Ascorbinsäure als Nitritfänger enthalten.

2. Die Zusammensetzung nach Anspruch 1, die eine granuläre Zusammensetzung ist; vorzugsweise Pellets.

3. Die Zusammensetzung nach einem der vorstehenden Ansprüche, wobei der Gesamtgewichtsanteil an Ascorbinsäure im Bereich von 0,05 bis 1,4 Gew.-%, vorzugsweise 0,1 bis 1,2 Gew.-%, noch bevorzugter 0,2 bis 1,0 Gew.-%, noch bevorzugter 0,4 bis 0,8 Gew.-%, am meisten bevorzugt 0,5 bis 0,7 Gew.-% und am allermeisten bevorzugt 0,55 bis 0,65 Gew.-% beträgt, jeweils bezogen auf das Gesamtgewicht der Zusammensetzung.

4. Die Zusammensetzung nach einem der vorstehenden Ansprüche, wobei das relative Verhältnis von in der Zwischenbeschichtungsschicht enthaltener Ascorbinsäure zu in der Wirkstoffbeschichtungsschicht enthaltener Ascorbinsäure im Bereich von 5,0:1 bis 29:1, vorzugsweise 7,0:1 bis 27:1, noch bevorzugter 9,0:1 bis 25:1, am meisten bevorzugt 11:1 bis 23:1, noch bevorzugter im Bereich von 13:1 bis 21:1 und am meisten bevorzugt im Bereich von 15:1 bis 19:1 liegt.

5. Die Zusammensetzung gemäß einem der vorstehenden Ansprüche, die einen Gehalt an N-Nitrosamin-Verunreinigungen, vorzugsweise N-Nitroso-Duloxetin, von höchstens 0,5 ppm aufweist, vorzugsweise höchstens 0,4 ppm, noch bevorzugter höchstens 0,3 ppm, am meisten bevorzugt höchstens 0,2 ppm, noch bevorzugter höchstens 0,1 ppm, am meisten bevorzugter höchstens 0,08 ppm, am meisten bevorzugter höchstens 0,06 ppm und insbesondere höchstens 0,04 ppm, jeweils bezogen auf das Gewicht (ppmw).

6. Die Zusammensetzung nach einem der vorstehenden Ansprüche, wobei der Kern Saccharose, Stärke, mikrokristalline Cellulose, pflanzliche Gummiarten, Wachse und Mischungen davon umfasst oder im Wesentlichen daraus besteht; vorzugsweise Saccharose; noch bevorzugter Saccharose-Pellets oder -Kügelchen.

7. Die Zusammensetzung nach einem der vorstehenden Ansprüche, die ein oder mehrere Bindemittel, ein oder mehrere Füllstoffe, ein oder mehrere Gleitmittel, ein oder mehrere magensaftresistente Polymere, ein oder mehrere Weichmacher und/oder Mischungen davon umfasst.

8. Die Zusammensetzung nach einem der vorstehenden Ansprüche, die ein oder mehrere magensaftresistente Polymere umfasst; vorzugsweise ausgewählt aus der Gruppe bestehend aus Methacrylsäure-Copolymeren, Celluloseacetatphthalat, Celluloseacetatsuccinat, Polymethacrylsäure, Hydroxypropylmethylcellulosephthalat, Hydroxypropylmethylcelluloseacetatsuccinat, Polyvinylacetatphthalat, Hydroxyethylethylcellulosephthalat, Celluloseacetattetrahydrophthalat und Mischungen davon; vorzugsweise Hydroxypropylmethylcellulosephthalat oder Hydroxypropylmethylcelluloseacetatsuccinat; noch bevorzugter Hydroxypropylmethylcelluloseacetatsuccinat.

9. Ein Verfahren zur Herstellung einer pharmazeutischen Zusammensetzung gemäß einem der vorstehenden Ansprüche, umfassend die folgenden Schritte:
(a) Herstellung einer Dispersion von Duloxetin oder eines physiologisch verträglichen Salzes davon, die vorzugsweise ein oder mehrere Bindemittel, ein oder mehrere Füllstoffe und Ascorbinsäure umfasst;
(b) Beschichten eines Kerns mit der in Schritt (a) bereitgestellten Dispersion;
(c) Herstellung einer Dispersion, die Ascorbinsäure, ein oder mehrere Bindemittel, ein oder mehrere Füllstoffe und/oder ein oder mehrere Gleitmittel enthält;
(d) Beschichten der in Schritt (b) erhaltenen Pellets mit der in Schritt (c) bereitgestellten Dispersion;
(e) Herstellung einer magensaftresistenten Dispersion, die ein oder mehrere magensaftresistente Polymere, ein oder mehrere Weichmacher und/oder ein oder mehrere Gleitmittel umfasst; wobei das eine oder die mehreren magensaftresistenten Polymere vorzugsweise zumindest teilweise neutralisiert sind;
(f) Beschichten der in Schritt (d) erhaltenen Pellets mit der in Schritt (e) bereitgestellten Dispersion;
(g) optional Herstellung einer Endbeschichtungsdispersion, die ein oder mehrere Bindemittel und/oder ein oder mehrere Pigmente umfasst; und
(h) optional Beschichten der in Schritt (f) erhaltenen Pellets mit der in Schritt (g) bereitgestellten Dispersion.

10. Das Verfahren nach Anspruch 9, wobei in Schritt (e) kein Aceton als Dispersionslösungsmittel verwendet wird; vorzugsweise kein organisches Lösungsmittel; noch bevorzugter wird Wasser als Dispersionslösungsmittel verwendet.

## Revendications

1. Composition pharmaceutique comprenant ou consistant essentiellement en:
- un noyau;
- une couche d'enrobage d'ingrédient actif comprenant de la duloxétine ou un sel physiologiquement acceptable de celle-ci, de préférence du chlorhydrate de duloxétine;
- une couche d'enrobage intermédiaire;
- une couche d'enrobage entérique; et
- éventuellement, une couche d'enrobage de finition;
dans laquelle la couche d'enrobage de principe actif et la couche d'enrobage intermédiaire comprennent de l'acide ascorbique en tant que piégeur de nitrites.

2. Composition selon la revendication 1, qui est une composition granulaire; de préférence des granulés.

3. Composition selon l'une quelconque des revendications précédentes, dans laquelle la teneur pondérale totale en acide ascorbique se situe dans la plage de 0,05 à 1,4 % en poids, de préférence de 0,1 à 1,2 % en poids, plus préférablement de 0,2 à 1,0 % en poids, encore plus de préférence de 0,4 à 0,8 % en poids, encore plus de préférence de 0,5 à 0,7 % en poids, et de préférence de 0,55 à 0,65 % en poids, dans chaque cas par rapport au poids total de la composition.

4. Composition selon l'une quelconque des revendications précédentes, dans laquelle le rapport relatif entre l'acide ascorbique contenu dans la couche d'enrobage intermédiaire et l'acide ascorbique contenu dans la couche d'enrobage du principe actif se situe dans la plage de 5,0:1 à 29:1, de préférence de 7,0:1 à 27:1, plus préférablement de 9,0:1 à 25:1, encore plus préférablement de 11:1 à 23:1, encore plus de préférence de 13:1 à 21:1, et de manière encore plus préférée de 15:1 à 19:1.

5. Composition selon l'une quelconque des revendications précédentes, qui présente une teneur en impureté de type N-nitrosamine, de préférence en N-nitroso-duloxétine, d'au plus 0,5 ppm, de préférence d'au plus 0,4 ppm, plus préférablement d'au plus 0,3 ppm, encore plus préférablement d'au plus 0,2 ppm, encore plus de préférence d'au plus 0,1 ppm, encore plus de préférence d'au plus 0,08 ppm, de préférence d'au plus 0,06 ppm, et en particulier d'au plus 0,04 ppm, dans chaque cas en poids (ppmw).

6. Composition selon l'une quelconque des revendications précédentes, dans laquelle le noyau comprend ou est essentiellement constitué de saccharose, d'amidon, de cellulose microcristalline, de gommes végétales, de cires et de mélanges de ceux-ci; de préférence de saccharose; de manière plus préférentielle de granulés ou de sphères de saccharose.

7. Composition selon l'une quelconque des revendications précédentes, qui comprend un ou plusieurs liants, un ou plusieurs agents de charge, un ou plusieurs lubrifiants, un ou plusieurs polymères gastro-résistants, un ou plusieurs agents plastifiants, et/ou des mélanges de ceux-ci.

8. Composition selon l'une quelconque des revendications précédentes, qui comprend un ou plusieurs polymères gastro-résistants; de préférence choisis parmi le groupe constitué des copolymères d'acide méthacrylique, de l'acétate-phtalate de cellulose, du succinate d'acétate de cellulose, de l'acide polyméthacrylique, du phtalate d'hydroxypropylméthylcellulose, du succinate d'acétate d'hydroxypropylméthylcellulose, du phtalate de poly(acétate de vinyle), du phtalate d'hydroxyéthyléthylcellulose, du tétrahydrophtalate d'acétate de cellulose, et de leurs mélanges; de préférence le phtalate d'hydroxypropylméthylcellulose ou le succinate d'acétate d'hydroxy-propylméthylcellulose; de manière plus préférentielle, le succinate d'acétate d'hydroxypropylmé-thylcellulose.

9. Procédé de préparation d'une composition pharmaceutique selon l'une quelconque des revendications précédentes, comprenant les étapes suivantes:
(a) préparer une dispersion de duloxétine ou d'un sel physiologiquement acceptable de celle-ci, comprenant de préférence un ou plusieurs liants, un ou plusieurs excipients et de l'acide ascorbique;
(b) enrobage d'un noyau avec la dispersion obtenue à l'étape (a);
(c) préparer une dispersion comprenant de l'acide ascorbique, un ou plusieurs liants, un ou plusieurs excipients et/ou un ou plusieurs lubrifiants;
(d) enrober les granulés obtenus à l'étape (b) avec la dispersion préparée à l'étape (c);
(e) préparer une dispersion entérique comprenant un ou plusieurs polymères gastro-résistants, un ou plusieurs agents plastifiants et/ou un ou plusieurs lubrifiants ; dans laquelle le ou les polymères gastro-résistants sont de préférence au moins partiellement neutralisés;
(f) enrober les granulés obtenus à l'étape (d) avec la dispersion préparée à l'étape (e);
(g) éventuellement, préparer une dispersion de finition comprenant un ou plusieurs liants et/ou un ou plusieurs pigments; et
(h) éventuellement, enduire les granulés obtenus à l'étape (f) avec la dispersion préparée à l'étape (g).

10. Procédé selon la revendication 9, dans lequel, à l'étape (e), on n'utilise pas d'acétone comme solvant de dispersion; de préférence, on n'utilise aucun solvant organique; plus préférablement, on utilise de l'eau comme solvant de dispersion.
